# EUROPEAN PATENT SPECIFICATION

(11) **EP 1 695 739 B1**
(45) Date of publication and mention of the grant of the patent: **18.08.2010**
(21) Application number: 06075427.2
(22) Date of filing: 24.02.2006
(51) Int. Cl.: A61N 5/10

(54) **Storage container**
Lagerbehälter
Récipient de stockage

(30) Priority: 28.02.2005 NL 1028428
(43) Date of publication of application: 30.08.2006
(73) Proprietor: Isodose Control Intellectual Property B.V., 3905 PE Veenendaal (NL)
(72) Inventor: van't Hooft, Eric, 2930 Brasschaat (BE)
(74) Representative: Winckels, Johannes Hubertus F.

(56) References cited:
- EP-A- 0 254 351
- US-A- 4 631 415
- US-A- 5 120 973
- US-A- 6 048 300

## Description

The invention relates to a storage container for storing radioactive sources.

From EP-A-0 128 300, an apparatus is known which is known as an afterloading apparatus for using brachytherapy. The apparatus comprises a storage safe in which a radioactive source is kept. Here, the source is mounted in a capsule of about 2 mm thick and 8 mm long, and is attached to the end of a new transport cable as one whole. By means of the cable, the source can be transported via a cable guide. By an actuator, the cable is moved from the safe through the cable guide and brought to a position to be irradiated in the human body via a catheter.

Introducing new sources and removing old, spent sources into and out of the transport apparatus involves considerable precautions to prevent irradiation. A new source is transported from a reprocessing center to a hospital or medical clinic with a storage unit specially developed for this purpose. The other end of the cable can be picked up from the storage unit by the afterloading apparatus and is wound up by the actuator, so that, finally, the source is moved from the storage unit and is introduced into the afterloading apparatus, in particular into the storage safe.

For the reverse process, the same storage unit is used to dispose a spent source therein and to discharge it. This roughly takes place by a reversal of steps: the source is guided into the transport medium via a cable guide, and the cable is then unwound until it is released. Then, the remaining cable is also inserted into the transport medium and the whole is closed off and is dispatched back.

The invention has recognized that this reverse process is cost-intensive because the spent source is each time returned individually.

Further, it is desired that the risk of radiation during transfer of the source into the storage unit and making it ready for transport be reduced further.

For this purpose, the invention provides a storage unit as intended in the opening paragraph, where a cutting clement is comprised for cutting off the cable above the radioactive source, so that the source is received in the safe and the remaining cable can be taken wherefrom.

For many years, the container can cut off spent sources from the source transport cable of an afterloading apparatus and store them for a prolonged period. It is an object of the apparatus to prevent the regular returning of spent radioactive sources so that a great saving in costs is achieved and the apparatus further has the advantage that less practiced users can send radioactive material that is not packaged according to the regulations. In particular, if the use of the medical radiation apparatus is ended after several years, the collected sources can even once be discharged to a company designated for this.

Therefore, in use, only the capsule needs to be kept in the storage unit. Since the capsule takes up very little space, the storage unit can be used more often and the unit need not be returned until after repeated use, for instance after a few years. This greatly reduces the costs for transport of spent sources.

In a preferred embodiment, a detector is provided in the storage unit for registering the storage. Thus, a reliable registration of spent sources can be maintained.

US5120973 is involved with storing radioactive sources that can be selectively released via a release means, which causes a needle-shaped holder filled with radio active material to be separated from traction wire. The publication relies on a releasable mechanism of a bush, pin and sleeve which introduces system complexities.

The invention will be explained in more detail with reference to the drawing, in which:
Fig. 1 shows a source storage system according to the prior art;
Fig. 2 shows a source storage system according to the invention; and
Fig. 3 shows a schematic view of the invention in conjunction with an afterloading apparatus.

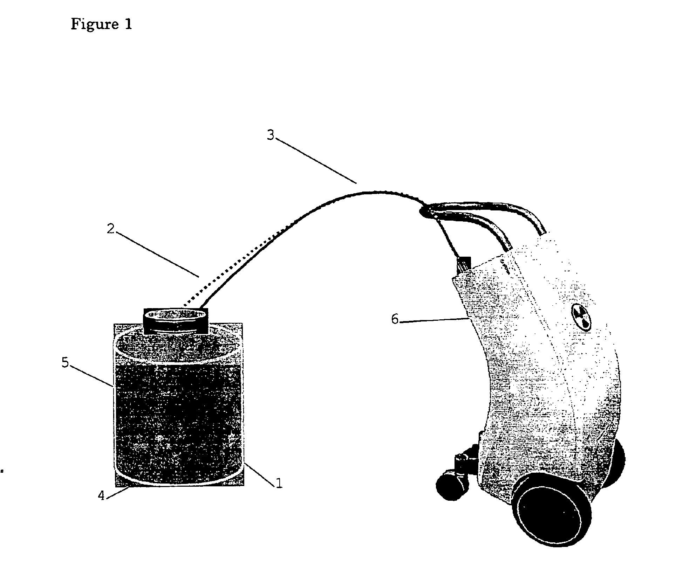

As Fig. 1 shows, a prior art is shown in which a radioactive source, such as for instance an IR¹⁹² source for irradiation of tumors, is stored in a safe 1. The safe 1 comprises an opening for introducing the end of a cable 2 (interrupted line) to which the source (not shown) has been attached. A cable guide 3 is attached to the storage unit 4 for guiding the cable 2 into the safe 1. With the same mechanism, a new source 5 (continuous line) is picked up from the safe 1 in order to be introduced into the afterloading apparatus 6. After the exchange of sources has taken place and the spent source has been disposed into the safe 1, the storage unit 4 is made ready and dispatched for further processing.

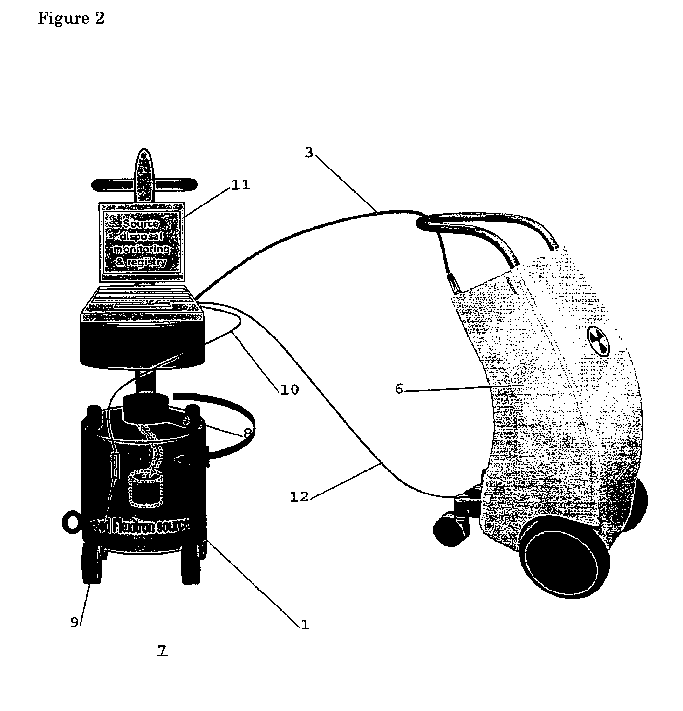

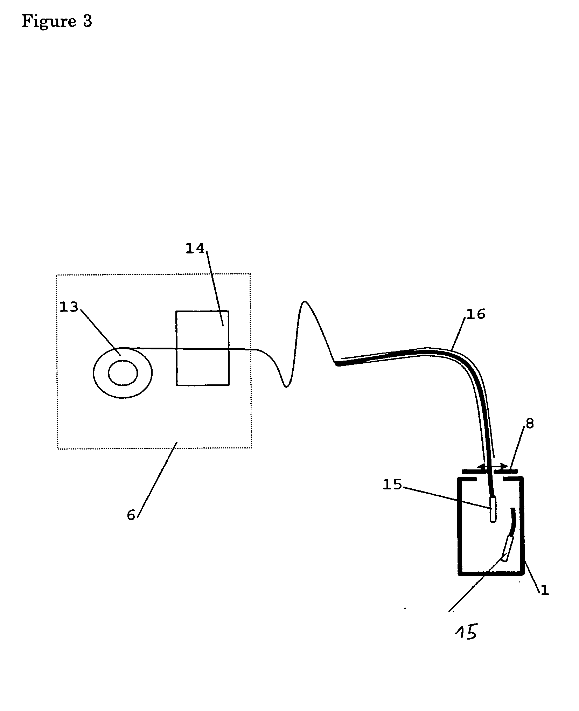

Fig. 2 shows storage system 7 for storing radioactive sources according to the invention. Here, it has been recognized that only the radioactive part of the cable 2, i.e. the capsule attached to the end thereof, needs to be kept, which offers advantages in terms of storage, transport, safety and manageability. Indeed, according to Fig. 2, a cable 2 can be inserted partly into the safe 1, so that the capsule is received by the safe 1. Then, the cable 2 can be cut through by the cutting element 8, so that a capsule (not shown) attached to the end of the cable 2 is separated. The cutting element 8 is provided with a safety unit, for instance in the form of a camera 9, which can register whether the cable 2 can be cut through safely. The cutting element is further designed in a known manner, for instance as a shearing blade (see Fig. 3) which can simultaneously serve as closure of the safe 1. The cutting element 8 can be driven manually or mechanically/electronically.

In addition to a visual check for correctly cutting through the cable 2, the camera 9 may also be arranged for forming a registration unit for registering the cutting through of the cable 2. For this purpose, the camera 9 has been connected (for instance by a USB-connection 10) with a computer 11 intended for that purpose. Accordingly, the camera 9 can serve for photographic registration with date indication.

A further data connection 12 may be provided for coupling of the afterloading apparatus 6 with the central computer 11 for exchange of data.

Fig. 3 again schematically shows the invention. An afterloading apparatus 6 is shown with a winding mechanism 13 therein for winding and unwinding a cable 2. In the apparatus 6, a safe 14 is included where the source 15 is kept when it is not used for irradiation. The winding mechanism 13 can move the cable into and out of the safe 14 via cable guide 16. A cutting element 8 cuts the source 15 from the end of the cable 2 and disposes it in the safe 1.

Although the invention has been explained with reference to the drawing, it is by no means limited thereto but may contain variations and modifications thereof without deviating from the scope of the invention. This scope is formed by the scope of the following claims.

## Claims

1. A storage unit (4) for storing radioactive sources (9) from an afterloading apparatus (6), wherein a radioactive source (9) has been attached to the end of a movable cable (2) for carrying out brachytherapy, which storage unit (4) comprises:
- a safe (1) provided with an opening for introducing the cable with the radioactive source; and
- an element (8) **characterised in that** the element is a cutting element (8) for cutting off the cable (2) above the radioactive source (9), so that the source is received in the safe (1) and the remaining cable (2) can be taken therefrom.

2. A storage unit (4) according to claim 1, **characterized in that** a safety unit (9) is provided for deactivating the cutting element to prevent cutting through the source.

3. A storage unit according to claim 2, **characterized in that** the safety unit (9) comprises a position sensor for registering the position of the source with respect to the safe.

4. A storage unit according to at least one of the preceding claims, **characterized in that** a registration unit (11) has been provided for registering the cutting through of the cable.

5. A storage unit according to claim 4, **characterized in that** the registration unit comprises a digital camera (9) which has been provided in the storage medium for providing a photographic registration with date indication.

6. A storage unit according to claim 5, **characterized in that** the digital camera (9) can be connected with a computer for storing the photographic registration.

7. A storage system for storing radioactive sources, comprising:
- a transport cart;
- a storage unit (4) according to at least one of the preceding claims held on a transport cart; and
- a computer (11) for providing a storage registration.

8. A method for storing radioactive sources from an afterloading apparatus, wherein a radioactive source has been attached to the end of a movable cable for carrying out brachytherapy, comprising:
- introducing the cable with the radioactive source into a safe (1); and
- cutting off the cable (2) above the radioactive source (9), so that the source is received in the safe and the remaining cable can be taken therefrom.

## Patentansprüche

1. Lagereinheit (4) zum Lagern radioaktiver Quellen (9) aus einer Afterloading-Vorrichtung (6), wobei eine radioaktive Quelle (9) an dem Ende eines beweglichen Seils (2) zum Ausführen von Brachytherapie befestigt ist, wobei die Lagereinheit (4) aufweist:
- Einen Sicherheitsbehälter (1), der mit einer Öffnung zum Einführen des Seils mit der radioaktiven Quelle versehen ist, und
- ein Element (8),
**dadurch gekennzeichnet, dass** das Element ein Schneidelement (8) zum Abschneiden des Seils (2) über der radioaktiven Quelle (9) ist, so dass die Quelle in dem Sicherheitsbehälter (1) aufgenommen ist und das übrige Seil (2) daraus entnommen werden kann.

2. Lagereinheit (4) nach Anspruch 1, **dadurch gekennzeichnet, dass** eine Sicherheitseinheit (9) vorgesehen ist, um das Schneidelement zu deaktivieren, um Schnitte durch die Quelle zu vermeiden.

3. Lagereinheit nach Anspruch 2, **dadurch gekennzeichnet, dass** die Sicherheitseinheit (9) einen Positionssensor zum Registrieren der Position der Quelle in Bezug auf den Sicherheitsbehälter aufweist.

4. Lagereinheit nach einem der vorhergehenden Ansprüche, **dadurch gekennzeichnet, dass** eine Registriereinheit (11) vorgesehen ist, um das Schneiden durch das Seil zu registrieren.

5. Lagereinheit nach Anspruch 4, **dadurch gekennzeichnet, dass** die Registriereinheit eine Digitalkamera (9) aufweist, die in dem Lagermedium vorgesehen ist, um eine photographische Registrierung mit einer Datumsanzeige zu liefern.

6. Lagereinheit nach Anspruch 5, **dadurch gekennzeichnet, dass** die Digitalkamera (9) mit einem Computer verbindbar ist, um die photographische Registrierung abzuspeichern.

7. Lagersystem zum Lagern radioaktiver Quellen, mit:
- Einem Transportwagen,
- einer Lagereinheit (4) nach wenigstens einem der vorhergehenden Ansprüche, die auf einem Transportwagen gehalten ist, und
- einem Computer (11) zum Bereitstellen einer Lagerungsregistrierung.

8. Verfahren zum Speichern radioaktiver Quellen aus einer Afterloading-Vorrichtung, wobei eine radioaktive Quelle an dem Ende eines beweglichen Seils zum Ausführen von Brachytherapie befestigt ist, bei dem
- das Seil mit der radioaktiven Quelle in einen Sicherheitsbehälter (1) eingeführt wird und
- das Seil (2) oberhalb der radioaktiven Quelle (9) abgeschnitten wird, so dass die Quelle in dem Sicherheitsbehälter aufgenommen ist und das verbleibende Seil daraus entnommen werden kann.

## Revendications

1. Récipient de stockage (4) pour stocker des sources radioactives (9) à partir d'un appareil d'introduction guidée (6) dans lequel une source radioactive (9) a été fixée à l'extrémité d'un câble mobile (2) pour effectuer une curiethérapie, ledit récipient de stockage (4) comprenant :
- un conteneur blindé (1) muni d'un orifice pour introduire le câble avec la source radioactive ; et
- un élément (8), **caractérisé en ce que** l'élément est un élément coupant (8) servant à couper le câble (2) au-dessus de la source radioactive (9) de telle façon que la source se loge dans le conteneur blindé (1) et le reste du câble (2) peut en être retiré.

2. Récipient de stockage (4) selon la revendication 1, **caractérisé en ce qu'**il est prévu un système de sécurité (9) pour désactiver l'élément coupant afin de l'empêcher de couper la source.

3. Récipient de stockage selon la revendication 2, **caractérisé en ce que** le système de sécurité (9) comprend un capteur de position pour détecter la position de la source par rapport au conteneur blindé.

4. Récipient de stockage selon l'une au moins des revendications précédentes, **caractérisé en ce qu'**il est prévu un système d'enregistrement (11) pour enregistrer la découpe du câble.

5. Récipient de stockage selon la revendication 4, **caractérisé en ce que** le système d'enregistrement comprend une caméra numérique (9) qui est prévue dans le milieu de stockage afin de permettre un enregistrement photographique avec indication de la date.

6. Récipient de stockage selon la revendication 5, **caractérisé en ce que** la caméra numérique (9) peut être reliée à un ordinateur afin de stocker l'enregistrement photographique.

7. Système de stockage de sources radioactives, comprenant :
- un chariot de transport ;
- un récipient de stockage (4) selon l'une au moins des revendications précédentes, fixé sur un chariot de transport ; et
- un ordinateur (11) servant à enregistrer le stockage.

8. Procédé de stockage de sources radioactives à partir d'un appareil d'introduction guidée dans lequel une source radioactive a été fixée à l'extrémité d'un câble mobile pour effectuer une curiethérapie, comprenant les étapes consistant à :
- introduire le câble avec la source radioactive dans un conteneur blindé (1) ; et
- couper le câble (2) au-dessus de la source radioactive (9) de telle façon que la source se loge dans le conteneur blindé et le reste du câble peut en être retiré.
